(19) Europäisches Patentamt
European Patent Office
Office européen des brevets

(11) EP 3 112 461 B1

(12) **EUROPEAN PATENT SPECIFICATION**

(45) Date of publication and mention of the grant of the patent:
**12.08.2020 Bulletin 2020/33**

(21) Application number: **15754649.0**

(22) Date of filing: **13.02.2015**

(51) Int Cl.:
*C12N 15/09* (2006.01) *C12M 1/40* (2006.01)
*C12N 1/15* (2006.01) *C12N 1/19* (2006.01)
*C12N 1/21* (2006.01) *C12N 5/10* (2006.01)
*C12N 9/04* (2006.01) *C12Q 1/32* (2006.01)
*C12Q 1/54* (2006.01)

(86) International application number:
**PCT/JP2015/053889**

(87) International publication number:
**WO 2015/129475 (03.09.2015 Gazette 2015/35)**

(54) **FLAVIN-BINDING GLUCOSE DEHYDROGENASE HAVING IMPROVED SPECIFIC ACTIVITY**

FLAVINBINDENDE GLUCOSEDEHYDROGENASE MIT VERBESSERTER SPEZIFISCHER AKTIVITÄT

GLUCOSE DÉSHYDROGÉNASE SE LIANT À LA FLAVINE AYANT UNE ACTIVITÉ SPÉCIFIQUE AMÉLIORÉE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **28.02.2014 JP 2014037737**

(43) Date of publication of application:
**04.01.2017 Bulletin 2017/01**

(73) Proprietor: **Kikkoman Corporation**
**Noda-shi, Chiba 278-8601 (JP)**

(72) Inventor: **ARAKI, Yasuko**
**Noda-shi**
**Chiba 278-8601 (JP)**

(74) Representative: **J A Kemp LLP**
**14 South Square**
**Gray's Inn**
**London WC1R 5JJ (GB)**

(56) References cited:
**EP-A1- 2 647 703**
**WO-A1-2012/169512**
**WO-A1-2013/065770**
**WO-A1-2013/065770**
**JP-A- 2010 035 448**
**JP-A- 2013 090 621**
**JP-B2- 4 648 993**

- **YAMAOKA H ET AL.: 'Site directed mutagenesis studies of FAD-dependent glucose dehydrogenase catalytic subunit of Burkholderia cepacia' BIOTECHNOL. LETT. vol. 30, no. 11, 2008, pages 1967 - 1972, XP019639462**
- **YAMASHITA Y ET AL.: 'Direct electron transfer type disposable sensor strip for glucose sensing employing an engineered FAD glucose dehydrogenase' ENZYME AND MICROBIAL TECHNOLOGY vol. 52, no. 2, 2013, ISSN 0141-0229 pages 123 - 128, XP055221039**
- **MORI K ET AL.: 'Screening of Aspergillus-derived FAD-glucose dehydrogenases from fungal genome database' BIOTECHNOL. LETT. vol. 33, no. 11, 2011, ISSN 1573-6776 pages 2255 - 2263, XP019957896**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 112 461 B1

## Description

### TECHNICAL FIELD

The present invention relates to a flavin-binding glucose dehydrogenase exhibiting improved specific activity, a method for measuring glucose using the same, and a method for producing a flavin-binding glucose dehydrogenase.

### BACKGROUND ART

Blood glucose concentration (blood glucose level) is an important marker for diabetes. Devices for self-monitoring of blood glucose (SMBG) employing an electrochemical biosensor are widely used by diabetes patients to monitor their own blood glucose levels. The biosensors used in SMBG devices have conventionally used an enzyme such as glucose oxidase (GOD) that uses glucose as a substrate. However, since GOD has the property of using oxygen as an electron acceptor, in SMBG devices using GOD, dissolved oxygen present in a measurement sample affects measured values, and cases can occur in which accurate measured values are unable to be obtained.

On the other hand, various types of glucose dehydrogenases (GDH) are known to be different enzymes that use glucose as a substrate but do not use oxygen as an electron acceptor. Specific examples of such enzymes that have been found include GDH (NAD(P)-GDH), which is a type that uses nicotinamide adenine dinucleotide (NAD) or nicotinamide adenine dinucleotide phosphate (NADP) as a coenzyme, and GDH (PQQ-GDH), which is a type that uses pyrroloquinoline quinone (PQQ) as a coenzyme, and these enzymes are used in the biosensors of SMBG devices. However, NAD(P)-GDH has the problems of lacking enzyme stability and requiring the addition of coenzyme, while PQQ-GDH has the problem of low substrate specificity, and as a result of acting on sugar compounds other than the glucose targeted for measurement such as maltose, D-galactose or D-xylose, allows sugar compounds other than glucose present in a measurement sample to have an effect on measured values, thereby preventing the obtaining of accurate measured values.

Cases have been reported in recent years in which, when blood sugar levels of diabetes patients receiving an infusion were measured using an SMBG device employing PQQ-GDH for the biosensor, the PQQ-GDH also acted on maltose contained in the infusion liquid resulting in the obtaining of measured values that were higher than the actual blood glucose levels, and causing the occurrence of hypoglycemia as a result of the patients being treated on the basis of those measured values. In addition, similar events have been determined to also have the potential to occur when performing galactose loading tests or xylose absorption tests (see, for example, Non-Patent Document 1). When, in response thereto, the Pharmaceutical and Food Safety Bureau of the Ministry of Health, Labour and Welfare conducted cross-reactivity tests for the purpose of investigating the effects on measured blood glucose values in the case of having added various sugars to glucose solutions, measured values obtained with a blood glucose assay kit using the PQQ-GDH method demonstrated values that were 2.5 to 3 times higher than the actual glucose concentrations in the case of having added maltose at 600 mg/dL, D-galactose at 300 mg/dL or D-xylose at 200 mg/dL. In other words, measured values were determined to be inaccurate due to maltose, D-galactose or D-xylose, which may be present in measurement samples, thereby resulting in the urgent desire to develop a GDH that is unaffected by such sugar compounds that cause measurement error and has a high level of substrate specificity that allows specific measurement of glucose.

In view of the circumstances described above, efforts have focused on GDH of a type that uses a coenzyme other than those described above. For example, although not describing details regarding substrate specificity, reports are known regarding GDH derived from Aspergillus oryzae (see, for example, Non-Patent Documents 2 to 5). In addition, glucose dehydrogenase (FAD-GDH) derived from Aspergillus species and Penicillium species has been disclosed that uses flavin adenine dinucleotide (FAD) as coenzyme (see, for example, Patent Documents 1 to 3), and FAD-GDH derived from Aspergillus species has been disclosed that reduces enzymatic action on D-xylose (see, for example, Patent Document 4).

However, although the aforementioned enzymes have the properties of demonstrating low reactivity with one or more sugar compounds that are not D-glucose, they do not have the property of having sufficiently low reactivity with maltose, D-galactose or D-xylose. In contrast, FAD-GDH discovered in a type of mold in the form of Mucor species was shown to have the superior property of having sufficiently low reactivity with maltose, D-galactose and D-xylose (see, for example, Patent Document 5). The use of this GDH makes it possible to accurately measure glucose concentration without being affected by maltose, D-galactose or D-xylose even under conditions in which these sugar compounds are present (see, for example, Patent Document 5). This superior substrate specificity is one of the characteristics that indicate the practical superiority of Mucor-derived FAD-GDH.

When considering the application of FAD-GDH to a blood glucose sensor, since there are cases in which the sensor chip production process includes a step of heat drying treatment, the FAD-GDH is generally required to have a high level of heat resistance. With this in mind, Patent Document 6 describes a modified Mucor-derived FAD-GDH having improved heat resistance. In addition, Patent Document 7 discloses a modified Mucor-derived FAD-GDH that demonstrates improved specificity by further decreasing reactivity with maltose and xylose.

Moreover, enzymes having even higher specific activity are sought in applications to blood glucose sensors. Improving specific activity makes it possible to improve reactivity on the sensor and complete measurements in a shorter period of time. In addition, since additional advantages such as cost savings stemming from reducing the amount of enzyme use and reduced measurement noise attributable to contaminants can also be expected, the development of an enzyme having high specific activity would be extremely industrially useful.

FAD-GDH with high substrate specificity for D-glucose is disclosed in Patent Document 8. FAD-GDH with improved substrate specificity is also disclosed in Patent Document 9.

Prior Art Documents

Patent Documents

Patent Document 1: Japanese Unexamined Patent Publication No. 2007-289148
Patent Document 2: Japanese Patent No. 4494978
Patent Document 3: International Publication No. WO 07/139013
Patent Document 4: Japanese Unexamined Patent Publication No. 2008-237210
Patent Document 5: Japanese Patent No. 4648993
Patent Document 6: International Publication No. WO 12/169512
Patent Document 7: International Publication No. WO 13/65770
Patent Document 8: EP 2 647 703 A1
Patent Document 9: JP 2010 035448

Non-Patent Documents

Non-Patent Document 1: Pharmaceuticals and Medical Devices Safety Information No. 206, October 2004, Pharmaceutical and Food Safety Bureau, Ministry of Health, Labour and Welfare
Non-Patent Document 2: Studies on the glucose dehydrogenase of Aspergillus oryzae: I. Induction of its synthesis by p-benzoquinone and hydroquinone, T.C. Bak and R. Sato, Biochim. Biophys. Acta, 139, 265-276 (1967)
Non-Patent Document 3: Studies on the glucose dehydrogenase of Aspergillus oryzae: II. Purification and physical and chemical properties, T.C. Bak, Biochim. Biophys. Acta, 139, 277-293 (1967)
Non-Patent Document 4: Studies on the glucose dehydrogenase of Aspergillus oryzae: III. General enzymatic properties, T.C. Bak, Biochim. Biophys. Acta, 146, 317-327 (1967)
Non-Patent Document 5: Studies on the glucose dehydrogenase of Aspergillus oryzae: IV. Histidyl residue as an active site, T.C. Bak and R. Sato, Biochim. Biophys. Acta, 146, 328-335 (1967)

DISCLOSURE OF THE INVENTION

Problems to be Solved by the Invention

An object of the present invention is to provide an FAD-GDH having improved specific activity.

Means for Solving the Problems

As a result of conducting extensive studies to solve the aforementioned problems, an FAD-GDH having improved specific activity was found to be obtained by introducing a mutation into a known FAD-GDH.

Namely, the present invention relates to that described below.

(1) An FAD-GDH consisting of the amino acid sequence represented by SEQ ID NO: 1, or an amino acid sequence having a sequence identity of 90% or more with the amino acid sequence represented by SEQ ID NO: 1, and, having one or more amino acid substitutions at positions corresponding to amino acids selected from the following (a) and/or (b), and having improved specific activity in comparison with prior to carrying out that substitution:

(a) the amino acid at position 88 in the amino acid sequence set forth in SEQ ID NO: 1,

(b) the amino acid at position 554 in the amino acid sequence set forth in SEQ ID NO: 1.

(2) The FAD-GDH according to (1) having an amino acid substitution selected from the following (c) and/or (d):

(c) the amino acid at the position corresponding to position 88 in the amino acid sequence set forth in SEQ ID NO: 1 is alanine,
(d) the amino acid at the position corresponding to position 554 in the amino acid sequence set forth in SEQ ID NO: 1 is aspartic acid.

(3) The FAD-GDH according to (2), wherein the amino acid at the position corresponding to the cysteine residue at position 88 is substituted with alanine.
(4) The FAD-GDH according to (2), wherein the amino acid at the position corresponding to the glutamic acid residue at position 554 is substituted with aspartic acid.
(5) The FAD-GDH according to (1), which has amino acid substitutions at the position corresponding to position 88 and the position corresponding to position 554 of SEQ ID NO: 1, and having improved specific activity in comparison with prior to carrying out those substitutions.
(6) The FAD-GDH according to (2), wherein
the amino acid at the position corresponding to position 88 in the amino acid sequence set forth in SEQ ID NO: 1 is alanine, and
the amino acid at the position corresponding to position 554 in the amino acid sequence set forth in SEQ ID NO: 1 is aspartic acid.
(7) The FAD-GDH according to (6) wherein the amino acid at the position corresponding to the cysteine residue at position 88 is substituted with alanine, and the amino acid at the position corresponding to the glutamic acid residue at position 554 is substituted with aspartic acid.
(8) An FAD-GDH gene encoding the FAD-GDH described in any of (1) to (7) above.
(9) Recombinant DNA comprising the FAD-GDH gene described in (8) above inserted into vector DNA.
(10) A host cell introduced with the recombinant DNA described in (9) above.
(11) A method for producing FAD-GDH, comprising the following steps:

(a) a step for culturing the host cell described in (10) above,
(b) a step for expressing the FAD-GDH gene contained in the host cell, and
(c) a step for isolating the FAD-GDH from the culture.

(12) A glucose measurement method using the FAD-GDH described in any of (1) to (7) above.
(13) A glucose assay kit containing the FAD-GDH described in any of (1) to (7) above.
(14) A glucose sensor containing the FAD-GDH described in any of (1) to (7) above.

Effects of the Invention

According to the present invention, FAD-GDH can be provided that has improved specific activity.

DESCRIPTION OF EMBODIMENTS

(Action Principle of FAD-GDH of Present Invention and

Method for Measuring Activity)

The FAD-GDH of the present invention catalyzes a reaction that forms glucono-δ-lactone by oxidizing the hydroxyl group of glucose in the presence of an electron acceptor.

This action principle can be used to measure the activity of the FAD-GDH of the present invention by using the following system, for example, that uses phenazine methosulfate (PMS) and 2,6-dichloroindophenol (DCIP) as electron acceptors.

(Reaction 1) D-glucose + PMS (oxidized form) → D- glucono-δ-lactone + PMS (reduced form)

(Reaction 2) PMS (reduced form) + DCIP (oxidized form) → PMS (oxidized form) + DCIP (reduced form)

In Reaction 1, PMS (reduced form) is formed accompanying oxidation of glucose. DCIP is then reduced accompanying oxidation of PMS (oxidized form) due to progression of the subsequent Reaction 2. By detecting the degree of disappearance of this DCIP (oxidized form) as an amount of change in optical absorbance at a wavelength of 600 nm, enzyme activity can be determined based on this amount of change.

Activity of the FAD-GDH of the present invention can be measured in accordance with the procedure indicated below. 2.05 mL of 100 mM phosphate buffer (pH 7.0), 0.6 mL of 1 M D-glucose solution and 0.15 mL of 2 mM DCIP solution are mixed and then warmed for 5 minutes at 37°C. Next, 0.1 mL of 15 mM PMS solution and 0.1 mL of enzyme sample solution are added to initiate the reaction. Optical absorbance is measured at the start of the reaction and over time, the amount of the decrease in optical absorbance at 600 nm (ΔA600) per minute accompanying progression of the enzyme reaction is determined, and GDH activity is calculated in accordance with the equation indicated below. At this time, 1 U of GDH activity is defined as the amount of enzyme that reduces 1 μmol of DCIP in one minute in the presence of D-glucose at a concentration of 200 mM at 37°C.

[Equation 1]

$$\text{GDH activity (U/mL)} = \frac{-(\Delta A600-\Delta A600_{blank}) \times 3.0 \times df}{16.3 \times 0.1 \times 1.0}$$

Furthermore, the value of 3.0 in the equation represents the amount of the reaction reagent and enzyme reagent (mL), 16.3 represents the millimolar molecular extinction coefficient ($cm^2/\mu mol$) under the present activity measurement conditions, 0.1 represents the amount of the enzyme solution (mL), 1.0 represents the cell path length (cm), ΔA600blank represents the amount of decrease in optical absorbance per minute at 600 nm in the case of starting the reaction by adding the buffer used for enzyme dilution instead of the enzyme sample solution, and df represents the dilution factor.

(Amino Acid Sequence of FAD-GDH of Present Invention)

The FAD-GDH of the present invention is characterized by being comprised of the amino acid sequence represented by SEQ ID NO: 1, or an amino acid sequence having a sequence identity of 90% or more with the amino acid sequence represented by SEQ ID NO: 1, preferably 95% or more, and having one or more amino acid substitutions at positions corresponding to the amino acids selected from position 88 or position 554 in the amino acid sequence set forth in SEQ ID NO: 1.

Preferably, an amino acid substitution at the position corresponding to position 88 in the FAD-GDH of the present invention is a substitution in which the amino acid at the position corresponding to the aforementioned position 88 is substituted with alanine, and an amino acid substitution at the position corresponding to position 554 is a substitution in which the amino acid at the position corresponding to the aforementioned position 554 is substituted with aspartic acid. Furthermore, in SEQ ID NO: 1, the amino acid at position 88 not having a substitution of the present invention is cysteine, and the amino acid at position 554 is glutamic acid.

In the FAD-GDH of the present invention, more preferable examples thereof include multiple mutants having a plurality of combinations of the aforementioned substitutions. Double mutants combining two of the aforementioned substitutions are included in the present invention. Accumulation of these mutations makes it possible to create FAD-GDH having more improved specific activity.

In addition, in creating multiple mutants as described above, substitutions at positions other than the positions of each of the types of substitutions described above can also be combined. In the case of introducing a single substitution, the position of the substitution may be that which does not demonstrate a remarkable effect in the manner of those at the aforementioned substitution sites, or may be that which demonstrates a synergistic effect as a result of introducing in combination with the aforementioned substitution sites.

In addition, the FAD-GDH of the present invention may also arbitrarily combine a mutation that improves substrate specificity, a mutation that improves heat stability, or a known mutation for the purpose of demonstrating a different effect, such as an effect that improves resistance to pH or a specific substance, in addition to a mutation that improves specific activity as described above. Even in cases in which a different type of mutation has been combined, such FAD-GDH is also included in the present invention provided it is able to demonstrate the effect of the present invention.

As will be subsequently described, the FAD-GDH of the present invention can be obtained by, for example, first acquiring a gene that encodes an amino acid sequence similar to the amino acid sequence of SEQ ID NO: 1 by an arbitrary method, and then introducing an amino acid substitution at any position that is equivalent to a prescribed position of SEQ ID NO: 1.

Examples of methods used to introduce a target amino acid substitution include methods in which a mutation is introduced randomly and methods in which a site-directed mutation is introduced at a presumed position. Examples of the former methods include the Error-Prone PCR Method (Techniques, 1, 11-15 (1989)) and methods using XL1-Red competent cells, in which errors easily occur during plasmid replication and which are susceptible to the occurrence of modifications during cell proliferation (Stratagene Corp.). In addition, examples of the latter methods include methods consisting of constructing a three-dimensional structure based on a crystal structure analysis of a target protein, selecting an amino acid predicted to yield a target effect based on that information, and introducing a site-directed mutation using the commercially available Quick Change Site-Directed Mutagenesis Kit (Stratagene Corp.) and the like. Alternatively, another example of the latter methods is a method consisting of selecting an amino acid predicted to yield a target effect by using the three-dimensional structure of a known protein having a high degree of homology with a target protein and introducing a site-directed mutation .

In addition, the "position corresponding to the amino acid sequence of SEQ ID NO: 1" referred to here, for example, refers to the same position in that alignment in the case of aligning the amino acid sequence of SEQ ID NO:1 with another FAD-GDH having an amino acid sequence having sequence identity with SEQ ID NO: 1 (90% or more, preferably 95% or more). Furthermore, amino acid sequence identity can be calculated by a program such as the GENETYX-Mac maximum matching and search homology programs (Software Development Corp.) or the DNASIS Pro maximum matching and multiple alignment programs (Hitachi Software Engineering Co., Ltd.).

In addition, an example of a method for specifying the "position corresponding to the amino acid sequence of SEQ ID NO: 1" referred to here, for example, can be carried out by comparing amino acid sequences using a known algorithm such as the Lipman-Pearson method, and imparting maximum identity to conserved amino acid residues present in the amino acid sequence of FAD-GDH. Aligning FAD-GDH amino acid sequences using such methods makes it possible to determine the positions of corresponding amino acids in each of the FAD-GDH sequences regardless of the presence of insertions or deletions in the amino acid sequences. Corresponding positions are thought to be present at the same positions in three-dimensional structures, and can be assumed to have similar effects with respect to the target FAD-GDH.

Although the FAD-GDH of the present invention is presumed to have various variations within the range of the aforementioned identity, these various FAD-GDHs can all be included in the FAD-GDH of the present invention provided they have the FAD-GDH activity described in the present description. FAD-GDH having such an amino acid sequence demonstrates high specific activity, thereby making it industrially useful.

In addition, it is important in the FAD-GDH of the present invention that the amino acid at the position corresponding to the aforementioned position 88 is alanine, or the amino acid at the position corresponding to aforementioned position 554 is aspartic acid, while it is not important as to whether or not they are the result of an artificial substitution procedure. For example, in the case of introducing a desired substitution into a protein such as the protein as set forth in SEQ ID NO: 1, in which the amino acids at the aforementioned positions are originally different from residues desired in the present invention, using a known technology, the desired amino acid residues are introduced by substitution. In contrast, in the case of acquiring a desired protein by a known total peptide synthesis, in the case of synthesizing an entire gene sequence so as to encode a protein having a desired amino acid sequence and acquiring a desired protein on the basis thereof, or in the case of a natural protein found to originally have such a sequence therein, the FAD-GDH of the present invention can be obtained without having to go through a step consisting of artificial substitution.

(Improvement of Specific Activity in FAD-GDH of Present

Invention)

Improvement of specific activity in the present invention is evaluated under conditions described in the activity measurement method described in the present description. Furthermore, the pH during specific activity measurement is 7.0 in the present description because the FAD-GDH of the present invention was developed for the purpose of measuring glucose in blood (blood glucose level) and the pH of the blood is in the vicinity of neutrality. Evaluating under conditions approximating actual use in this manner makes it possible to acquire a more useful enzyme.

The FAD-GDH of the present invention is characterized in that specific activity is improved under the reaction conditions described in the activity measurement method described in the present description, in comparison with FAD-GDH in which amino acids of the specific positions described in the present invention are not the specific amino acids of the present invention. More specifically, the improvement in specific activity is characterized in that, when a value of 100 is assigned to the specific activity of FAD-GDH in which the amino acids of specific positions described in the present invention are not the specific amino acids of the present invention, the improved specific activity is greater than 100, preferably 105 or more, more preferably 110 or more and even more preferably 120 or more.

In addition, in addition to improving specific activity as previously described, the FAD-GDH of the present invention is more preferably also provided with performance more suitable for actual use with respect to other enzymatic properties as well. For example, the ratio of reactivity with D-xylose to reactivity with D-glucose (Xyl/Glc(%)) and/or the ratio of reactivity with maltose to reactivity with D-glucose (Mal/Glc(%)) are preferably 2% or less. For example, heat stability is preferably such that residual activity after heat treatment for 15 minutes at 40°C and pH 7.0 is 50% or more, preferably 60% or more and more preferably 70% or more. For example, the Km value is preferably 100 mM or less and more preferably 90 mM or less. These properties are preferable from the viewpoint of the performance typically required of FAD-GDH, and among the FAD-GDH of the present invention, FAD-GDH also provided with other such preferable properties typically required have particularly high industrial value.

(Acquisition of Gene Encoding FAD-GDH of Present

Invention)

A method utilizing genetic engineering techniques is preferably used to efficiently acquire the FAD-GDH of the present invention. An ordinary, commonly used genetic cloning method may be used to acquire a gene encoding the FAD-GDH of the present invention (to be referred to as FAD-GDH gene). For example, in order to acquire the FAD-GDH of the present invention by modifying a known FAD-GDH, chromosomal DNA or mRNA can be extracted from known microbial cells or various other cells having the ability to produce FAD-GDH according to an ordinary method such as the method described in Current Protocols in Molecular Biology, Wiley Interscience (1989)). Moreover, cDNA can be synthesized by using mRNA as a template. A chromosomal DNA or cDNA library can then be prepared using chromosomal DNA or cDNA obtained in this manner.

Next, DNA containing the total length of a target FAD-GDH gene can be obtained by amplifying DNA containing target gene fragments encoding FAD-GDH having high specific activity and then linking these DNA fragments by a method in which a suitable probe DNA is synthesized based on amino acid sequence information of a known FAD-GDH followed by using this probe DNA to select an FAD-GDH gene having high specific activity from a chromosomal DNA or cDNA library, or a suitable polymerase chain reaction method (PCR method) such as 5'RACE or 3'RACE by preparing suitable primer DNA based on the aforementioned amino acid sequence.

A method consisting of selecting FAD-GDHs using as indices enzymological properties of FAD-GDHs expressed from various mutant genes obtained by introducing a mutation into a known gene encoding FAD-GDH can be used as a method for acquiring the FAD-GDH of the present invention having superior specific activity by modifying a known FAD-GDH.

Any known method corresponding to an intended mutant form can be carried out for mutation treatment on a known FAD-GDH gene. Namely, a widely used method can be used, such as a method consisting of contacting a chemical agent serving as a mutagen with an FAD-GDH gene or recombinant DNA incorporating that gene and allowing it to act thereon, an ultraviolet radiation method, a genetic engineering method or a method that employs a protein engineering technique.

Examples of chemical agents used as mutagens in the aforementioned mutation treatment include hydroxylamine, N-methyl-N'-nitro-N-nitrosoguanidine, nitrous acid, sulfurous acid, hydrazine, formic acid and 5-bromouracil.

Conditions corresponding to the type of chemical agent used and the like can be employed for the various conditions for this contact and action, and there are no particular limitations thereon provided a desired mutation can actually be induced in various types of known FAD-GDH genes. Normally, a desired mutation can be induced by contacting and allowing to act for 10 minutes or longer, and preferably for 10 minutes to 180 minutes, at a concentration of the aforementioned chemical agent of 0.5 M to 12 M and at a reaction temperature of 20°C to 80°C. In the case of irradiating with ultraviolet light as well, irradiation can be carried out in accordance with an ordinary method as previously described (Chemistry Today, 24-30, June 1989).

A technique known as site-specific mutagenesis can typically be used as a method that employs a genetic engineering technique. Examples thereof include the Kramer method (Nucleic Acids Res., 12, 9441 (1984); Methods Enzymol., 154, 350 (1987); Gene, 37, 73 (1985)), the Eckstein method (Nucleic Acids Res., 13, 8749 (1985); Nucleic Acids Res., 13, 8765 (1985); Nucleic Acids Res., 14, 9679 (1986)), and the Kunkel method (Proc. Natl. Acad. Sci. U.S.A., 82, 488 (1985); Methods Enzymol., 154, 367 (1987)). Specific examples of methods for transforming a base sequence present in DNA include the use of a commercially available kit (such as the Transformer Mutagenesis Kit (Clontech Laboratories, Inc.), ExOIII/Mung Bean Deletion Kit (Stratagene Corp.) or Quick Change Site-Directed Mutagenesis Kit (Stratagene Corp.)).

In addition, a technique commonly known as a polymerase chain reaction can also be used (Technique, 1, 11 (1989)).

Furthermore, in addition to the aforementioned genetic modification methods, a modified FAD-GDH gene having desired superior heat stability can be synthesized directly by an organic synthesis method or enzymatic synthesis method.

The Multi-Capillary DNA Analysis System CEQ2000 (Beckman Coulter Inc.), for example, may be used in the case of determining or confirming the DNA base sequence of the FAD-GDH gene of the present invention selected according to any of the methods described above.

(Examples of Naturally-Occurring FAD-GDH Serving as

Source of FAD-GDH of Present Invention)

The FAD-GDH of the present invention can also be acquired by modifying a known FAD-GDH or known modified forms thereof. Preferable examples of known microorganisms serving as sources of FAD-GDH include microorganisms classified as members of the subphylum Mucoromycotina, preferably members of the class Mucoromycetes, more preferably members of the order Mucorales, and even more preferably members of the family Mucoraceae. Specific examples include FAD-GDH derived from Mucor species, Absidia species, Actinomucor species and Circinella species.

Specific preferable examples of microorganisms classified as Mucor species include Mucor prainii, Mucor javanicus, Mucor circinelloides f. circinelloides, Mucor guilliermondii, Mucor hiemalis f. silvaticus, Mucor subtilissimus and Mucor dimorphosporous. More specifically, examples include the Mucor prainii, Mucor javanicus, and Mucor circinelloides f. circinelloides described in Patent Document 5, and Mucor guilliermondii NBRC9403, Mucor hiemalis f. silvaticus NBRC6754, Mucor subtilissimus NBRC6338, Mucor RD056860 and Mucor dimorphosporous NBRC5395. Specific preferable examples of microorganisms classified as Absidia species include Absidia cylindrospora and Absidia hyalospora. More specifically, examples include the Absidia cylindrospora and Absidia hyalospora described in Patent Document 5. Specific preferable examples of microorganisms classified as Actinomucor species include Actinomucor elegans. More specific examples include the Actinomucor elegans described in Patent Document 5. Specific preferable examples of microorganisms classified as Circinella species include Circinella minor, Circinella mucoroides, Circinella muscae, Circinella rigida, Circinella simplex and Circinella umbellata. More specific examples include Circinella minor NBRC6448, Circinella mucoroides NBRC4453, Circinella muscae NBRC6410, Circinella rigida NBRC6411, Circinella simplex NBRC6412, Circinella umbellate NBRC4452, Circinella umbellata NBRC5842, Circinella RD055423 and Circinella RD055422. Furthermore, NBRC strains and RD strains refer to stock strains of the Biological Resource Center of the National Institute of Technology and Evaluation (NBRC).

(Vectors and Host Cells Inserted with FAD-GDH Gene of

Present Invention)

An FAD-GDH gene of the present invention obtained in the manner described above can be incorporated in a vector such as a bacteriophage, cosmid or plasmid used to transform prokaryotic cells or eukaryotic cells in accordance with ordinary methods followed by transforming or transducing host cells corresponding to each vector in accordance with ordinary methods.

Examples of prokaryotic host cells include microorganisms belonging to the genus Escherichia such as Escherichia coli strain K-12, Escherichia coli BL21(DE3), Escherichia coli JM109, Escherichia coli DH5$\alpha$, Escherichia coli W3110 or Escherichia coli C600 (all available from Takara Bio Inc.). These cells can then be transformed or transduced to obtain host cells introduced with DNA (transformants). A method for transferring recombinant DNA in the presence of calcium ions can be used to transfer a recombinant vector into such host cells in the case, for example, the host cells are microorganisms belonging to the genus Escherichia, and moreover, electroporation may also be used. Commercially available competent cells (such as ECOS Competent Escherichia coli BL21(DE3), Nippon Gene Co., Ltd.) may also be used.

An example of eukaryotic host cells is yeast. Examples of microorganisms classified as yeast include yeast belonging to the genus Zygosaccharomyces, genus Saccharomyces, genus Pichia and genus Candida. Inserted genes may contain marker genes for enabling selection of transformed cells. Examples of marker genes include genes that complement the nutritional requirements of the host in the manner of URA3 or TRP1. In addition, the inserted gene preferably also contains a promoter, enabling a gene of the present invention to be expressed in host cells, or other control sequences (such as an enhancer sequence, terminator sequence or polyadenylation sequence). Specific examples of promoters include the GAL1 promoter and ADH1 promoter. Although a known method such as a method using lithium acetate (Methods Mol. Cell. Biol., 5, 255-269 (1995)) or electroporation (J. Microbiol. Methods, 55, 481-484 (2003)) can be preferably used to transform yeast, the method used is not limited thereto, but rather transformation may be carried out using various types of arbitrary techniques such as the spheroplast method or glass bead method.

Other examples of eukaryotic host cells include mold cells in the manner of Aspergillus species and Tricoderma species. The inserted gene preferably contains a promoter, enabling a gene of the present invention to be expressed in host cells (such as the tef1 promoter), and other control sequences (such as a secretion signal sequence, enhancer sequence, terminator sequence or polyadenylation sequence). In addition, the inserted gene may also contain a marker gene such as niaD or pyrG for enabling the selection of transformed cells. Moreover, the inserted gene may also contain a homologous recombination domain for inserting into an arbitrary chromosome site. A known method such as a method using polyethylene glycol and calcium chloride following protoplast formation (Mol. Gen. Genet., 218, 99-104 (1989)) can be preferably used to transform molds.

(Production of FAD-GDH of Present Invention)

The FAD-GDH of the present invention may be produced by culturing host cells that produce the FAD-GDH of the present invention acquired in the manner described above, expressing the FAD-GDH gene contained in the host cells, and then isolating FAD-GDH from the culture.

Examples of media used to culture the aforementioned host cells include that obtained by adding one or more types of inorganic salts such as sodium chloride, potassium dihydrogen phosphate, dipotassium hydrogen phosphate, magnesium sulfate, magnesium chloride, ferric chloride, ferric sulfate or manganese sulfate to one or more types of nitrogen sources such as yeast extract, tryptone, peptone, beef extract, corn stiplica or soybean or wheat bran exudate, and further suitably adding carbohydrate raw materials or vitamins and the like as necessary.

Although there are no particular limitations thereon, the initial pH of the medium can be adjusted to, for example pH 5 to 9.

The culturing temperature is 10°C to 42°C, and culturing is preferably carried out at about 25°C. For example, culturing may be carried out by aeration-agitation submerged culturing, shake culturing or static culturing and the like for 4 hours to 24 hours in the case of prokaryotic cells or for 1 day to 10 days in the case of eukaryotic cells.

Following completion of culturing, the FAD-GDH of the present invention is harvested from the culture. An ordinary known enzyme harvesting means may be used. For example, microbial cells can be subjected to ultrasonic pulverization treatment or grinding treatment in accordance with ordinary methods, the enzyme can be extracted using a lytic enzyme such as lysozyme, or the enzyme can be expelled outside the microbial cells by lysing the cells by shaking or allowing to stand in the presence of toluene and the like. A crude FAD-GDH of the present invention is then obtained by filtering this solution, removing the solid fraction by centrifugal separation, removing nucleic acids with streptomycin hydrochloride, protamine sulfate or manganese sulfate and the like as necessary, followed by fractionating by addition of ammonium sulfate, alcohol or acetone and the like and harvesting the precipitate.

The crude FAD-GDH enzyme of the present invention can be further purified using any known means. A purified FAD-GDH enzyme preparation of the present invention can be obtained by, for example, suitably selecting a gel filtration method using Sephadex, Ultrogel or Biogel, an adsorption elution method using an ion exchanger, an electrophoresis method using polyacrylamide gel, an adsorption elution method using hydroxyapatite, a precipitation method such as sucrose density gradient centrifugation, an affinity chromatography method, or a fractionation method using a molecular sieve membrane or hollow fiber membrane, or using a combination thereof.

(Method for Measuring Glucose using FAD-GDH of Present

Invention)

The present invention also provides a glucose assay kit that contains the FAD-GDH of the present invention, and glucose in the blood (blood glucose level) can be measured using the FAD-GDH of the present invention.

The glucose assay kit of the present invention contains an amount of FAD-GDH modified in accordance with the present invention sufficient for at least one assay. Typically, the glucose assay kit of the present invention also contains a buffer, mediator and glucose standard solutions for preparing a calibration curve required for the assay in addition to the modified FAD-GDH of the present invention. The modified FAD-GDH used in the glucose measurement method and glucose assay kit of the present invention can be provided in various forms, such as in the form of a freeze-dried reagent or dissolved in a suitable preservative solution.

Measurement of glucose concentration can be carried out in the manner indicated below, for example, in the case of a colorimetric glucose assay kit. A liquid or solid composition containing FAD-GDH, an electron acceptor and one or more reaction accelerators in the form of substances selected from the group consisting of N-(2-acetamido)iminodiacetic acid (ADA), bis(2-hydroxyethyl)iminotris(hydroxymethyl)methane (Bis-Tris), sodium carbonate and imidazole is retained in the reaction layer of the glucose assay kit. Here, a pH buffer or coloring reagent is added as necessary. A sample containing glucose is then added thereto and allowed to react for a fixed period of time. During this time, optical absorbance corresponding to the maximum absorption wavelength of an electron acceptor that is discolored by reduction, or a pigment formed by polymerization as a result of accepting electrons from an electron acceptor, is monitored. Glucose concentration in the sample can be calculated on the basis of a calibration curve prepared in advance using glucose solutions having standard concentrations, from the rate of change in absorbance per unit time if using a rate method, or from the change in absorbance to the point all glucose in the sample has been oxidized if using the endpoint method.

In the case of using a mediator and coloring reagent able to be used in this method, glucose can be quantified by adding 2,6-dichlorophenolindophenol (DCPIP) as an electron acceptor followed by monitoring the decrease in absorbance at 600 nm. In addition, glucose concentration can be calculated by adding an electron acceptor in the form of phenazine methosulfate (PMS) and a coloring reagent in the form of nitrotetrazolium blue (NTB) followed by determining the amount of diformazan formed by measuring absorbance at 570 nm. Furthermore, it goes without saying that the electron acceptors and coloring reagents used are not limited thereto.

(Glucose Sensor Containing FAD-GDH of Present Invention)

The present invention also provides a glucose sensor that uses the FAD-GDH of the present invention. An electrode such as a carbon electrode, gold electrode or platinum electrode is used for the electrode, and the FAD-GDH of the present invention is immobilized on this electrode. Examples of immobilization methods include a method using a crosslinking agent, a method consisting of enclosing in a polymer matrix, a method consisting of coating with a dialysis membrane and methods using a photocrosslinkable polymer, electrically conductive polymer and oxidation-reduction polymer, or the FAD-GDH may be immobilized in a polymer together with an electrode mediator represented by ferrocene or a derivative thereof, immobilized by adsorbing to the electrode, or a combination of these methods may be used. Typically, the FAD-GDH of the present invention is immobilized on a carbon electrode using glutaraldehyde followed by treating with a reagent having an amino group to block the glutaraldehyde.

Glucose concentration can be measured in the manner indicated below. A buffer is placed in a thermostatic cell and maintained at a constant temperature. Potassium ferricyanide or phenazine methosulfate, for example, can be used for the mediator. The electrode having the modified FAD-GDH of the present invention immobilized thereon is used for the working electrode, and a counter electrode (such as a platinum electrode) and reference electrode (such as an Ag/AgCl electrode) are also used. A constant voltage is applied to the carbon electrode, and after the current has reached a steady state, a sample containing glucose is added followed by measurement of the increase in current. The glucose concentration in the sample can then be calculated in accordance with a calibration curve prepared with glucose concentrations having standard concentrations.

As a specific example thereof, 1.5 U of the FAD-GDH of the present invention are immobilized on a glassy carbon (GC) electrode and the response current value with respect to glucose concentration is measured. 1.8 ml of 50 mM potassium phosphate buffer (pH 6.0) and 0.2 ml of a 1 M aqueous solution of potassium hexacyanoferrate (III) (potassium ferricyanide) are added to an electrolysis cell. The GC electrode is connected to a BAS100B/W potentiostat (BAS Co., Ltd.) and the solution is stirred at 37°C followed by applying a voltage of + 500 mV to the silver/silver chloride reference electrode. Glucose solutions having final concentrations of 5 mM, 10 mM, 20 mM, 30 mM, 40 mM and 50 mM are added to these systems followed by measurement of the steady-state current value for each addition. These current values are plotted versus known glucose concentrations (5 mM, 10 mM, 20 mM, 30 mM, 40 mM, 50 mM) to prepare a calibration curve. As a result, glucose can be quantified with an enzyme-immobilized electrode using the FAD-binding glucose dehydrogenase of the present invention.

The following provides a more detailed explanation of the present invention through examples thereof. However, the technical scope of the present invention is not limited in any way by these examples.

Examples

In the present invention, evaluations of specific activity and substrate specificity of modified FAD-GDH were carried out in accordance with the methods of the test example indicated below unless specifically mentioned otherwise.

[Test Example]

(1) Preparation of Yeast Transformants Expressing Various

Types of Modified FAD-GDH

A recombinant plasmid (pYES2C-Mp (wild type)) that encodes FAD-GDH gene derived from Mucor prainii (wild-type MpGDH gene) of SEQ ID NO: 2 was acquired in compliance with the method described in Patent Document 7.

PCR reactions were carried out under the conditions indicated below using KOD-Plus- (Toyobo Co., Ltd.) and synthetic nucleotides for introducing various amino acid substitutions using the resulting recombinant plasmid pYE2C-Mp as template.

In other words, 5 μl of 10xKOD-Plus- buffer, 5 μl of a dNTPs mixed solution prepared so that the concentration of each dNTP was 2 mM, 2 μl of 25 mM $MgSO_4$, 50 ng of pYE2C-Mp serving as template, 15 pmol of each of the aforementioned synthetic oligonucleotides, and 1 unit of KOD-Plus- were added followed by adding sterilized water to a total volume of 50 μl to prepare a reaction solution. The prepared reaction solution was incubated for 2 minutes at 94°C using a thermal cycler (Eppendorf AG) followed by repeating 30 cycles consisting of 15 seconds at 94°C, 30 seconds at 55°C and 8 minutes at 68°C.

A portion of the reaction solution treated in the manner described above was subjected to electrophoresis with 1.0% agarose gel to confirm that about 8 kbp of DNA had been specifically amplified. The amplified DNA was treated with restrictase DpnI (New England Biolabs Inc.) followed by carrying out transformation by mixing with competent cells of E. coli strain JM109 (Nippon Gene Co., Ltd.) in accordance with the protocol provided. Next, the acquired transformants were respectively applied to LB-amp agar media and cultured. The colonies that formed were inoculated into LB-amp culture broth, and subjected to shake culturing followed by isolating various types of plasmid DNA containing about 8 kbp of amplified DNA (such as pYE2C-Mp-C88A or pYE2C-Mp-E554D in Example 1) in accordance with the protocol provided. Next, the base sequences of DNA encoding MpGDH gene in each of these plasmid DNAs were determined using the Multi-Capillary DNA Analysis System CEQ2000 (Beckman Coulter Inc.), and amino acids were confirmed to be substituted at the prescribed positions in the amino acid sequence set forth in SEQ ID NO: 1. In this manner, yeast expression vectors pYE2C-Mp (modified types, such as pYE2C-Mp-C88Aor pYE2C-Mp-E554D in Example 1) were acquired that encode modified MpGDH having prescribed amino acid substitutions.

Subsequently, pYE2C-Mp (wild type) and various types of mutation-introduced pYES2C-Mp (modified types, such as pYE2C-Mp-C88A or pYE2C-Mp-E554D in Example 1) were transformed in strain Inv-Sc (Invitrogen Corp.) using an S. cerevisiae transformation kit (Invitrogen Corp.) to respectively acquire yeast transformant strain Sc-Mp (wild type) expressing wild-type MpGDH and yeast transformant strains Sc-Mp expressing various types of modified MpGDH (modified types, such as Sc-Mp-C88A and Sc-Mp-E554D in Example 1).

(2) Purification and Measurement of Specific Activity of

Various Types of Yeast-Expressed FAD-GDH

The yeast transformant strain Sc-Mp (wild type) and various yeast transformant strains Sc-Mp (modified type, such as Sc-Mp-C88A and Sc-Mp-E554D in Example 1) were respectively cultured for 24 hours at 30°C in 10 mL of pre-culturing broth (consisting of 0.67% (w/v) yeast nitrogen base without amino acids (Becton, Dickinson & Co.), 0.192% (w/v) yeast synthetic drop-out medium supplement without uracil (Sigma Corp.) and 2.0% (w/v) raffinose). Subsequently, 10 ml of pre-culturing broth was added to 40 mL of final culturing broth (consisting of 0.67% (w/v) yeast nitrogen base without amino acids, 0.192% (w/v) yeast synthetic drop-out medium supplement without uracil, 2.5% (w/v) D-galactose and 0.75% (w/v) raffinose) followed by culturing for 14 hours at 30°C. The culture broth was then centrifuged (10,000 $\times$ g, 4°C, 3 minutes) to separate the microbial cells and culture supernatant, after which the culture supernatant was confirmed to have GDH activity and then used in the following procedure.

The recovered culture supernatant containing the FAD-GDH targeted for evaluation was concentrated with a centrifugal filter unit (Amicon Ultra-15 30K, Merck Millipore Corp.) followed by replacing with 20 mM potassium phosphate buffer (pH 6.0).

Next, the recovered concentrate was applied to a Superdex 200 10/300GL column (GE Healthcare Biosciences Corp.) preliminarily equilibrated with 20 mM potassium phosphate buffer containing 150 mM sodium chloride followed by recovery of the active fraction to acquire the purified enzyme.

The specific activity of the purified enzyme was measured using the previously described GDH activity measurement method based on activity per absorbance (U/A280) at 280 nm (A280). The "relative specific activity" in the modified type of MpGDH was calculated based on a value of 100 for the specific activity of wild-type MpGDH. The specific activity of the modified MpGDH was determined to have improved when relative specific activity was greater than 100.

(3) Evaluation of Substrate Specificity

The "ratio of reactivity with D-xylose to reactivity with D-glucose (Xyl/Glc(%))" was calculated by measuring activity by changing the substrate of the aforementioned activity measurement method from D-glucose to a system containing the same molar concentration of D-xylose using the purified enzyme acquired in the method described in (2). This value is the value obtained by dividing FAD-GDH activity when measured using D-xylose as substrate by FAD-GDH activity when measured using D-glucose as substrate and is represented as a percentage.

Example 1

(Evaluation of Specific Activity and Substrate

Specificity of Modified Types of MpGDH)

PCR reactions were carried out on the combinations of synthetic nucleotides having the sequence ID numbers shown in Table 1 using pYE2C-Mp (wild type) as template plasmid in accordance with the method described in the previous test example. Next, E. coli strain JM109 was transformed using a vector containing amplified DNA, and by determining the base sequence of DNA encoding MpGDH in the plasmid DNA retained by the colonies that formed, recombinant plasmids in the form of pYE2C-Mp-C88A and pYE2C-Mp-E554D were respectively acquired in which cysteine at position 88 of the amino acid sequence set forth in SEQ ID NO: 1 was substituted with alanine and glutamic acid at position 554 was substituted with aspartic acid.

Next, strain Inv-Sc was transformed and the acquired transformant strains (strain Sc-Mp-C88A and strain Sc-Mp-E554D) were cultured and purified in accordance with sections (1) and (2) of the test example using recombinant plasmids pYE2C-Mp-C88A and pYE2C-Mp-E554D encoding each modified type of MpGDH introduced with a site-directed mutation, followed by measuring specific activity (U/A280) and then calculating relative specific activity.

Continuing, the ratio of reactivity with D-xylose to reactivity with D-glucose (Xyl/Glc(%)) was measured based on the procedure of section (3) of the aforementioned test example.

Furthermore, in Table 1, for example, "C88A" means that C (Cys) at position 88 is substituted with A (Ala).

[Table 1]

| Recombinant Plasmid | Primer Sequence Nos. | Relative Specific Activity | Xyl/Glc (%) |
|---|---|---|---|
| pYE2C-Mp (wild-type, comparative example) | - | 100 | 1.4 |
| pYE2C-Mp-C88A (present invention) | 3, 4 | 119 | 1.2 |
| pYE2C-Mp-E554D (present invention) | 5, 6 | 107 | 1.4 |

As shown in Table 1, specific activity was confirmed to improve as a result of site-directed mutagenesis at position 88 or 554 in the wild-type MpGDH of SEQ ID NO: 1, and more specifically, as a result of introducing a site-directed mutation of C88A or E554D. More specifically, specific activity improved to 119 in the case of C88A and to 107 in the case of E554D in the case of a value of 100 for the specific activity in the wild-type serving as a comparative example.

Furthermore, the modified types of FAD-GDH having improved specific activity were confirmed to not only demonstrate improved specific activity, but also have a favorable ratio of Xyl/Glc (%). Although the wild-type FAD-GDH of the present example is an enzyme that has high substrate specificity in which the ratio of Xyl/Glc (%) is lower than 2%, in the case of having introduced a mutation that improves specific activity of the present invention, specific activity was determined to be maintained without impairing this favorable substrate specificity. In the case of C88A in particular, specific activity not only improved in comparison with the wild-type, but substrate specificity was also confirmed to be further improved, thereby demonstrating that this mutation is a mutation that is highly useful for improving the performance of FAD-GDH.

Example 2

(Study on Combined Mutation Introduction)

Next, mutants were prepared having both the mutations of C88A and E554D followed by evaluations of their specific activity and substrate specificity. More specifically, PCR reactions were carried out on the combinations of synthetic nucleotides of SEQ ID NOs: 5 and 6 using pYE2C-Mp-C88A as a template plasmid in accordance with the method described in the aforementioned test example. Next, E. coli strain JM109 was transformed using a vector containing the amplified DNA, and by determining the base sequence of DNA encoding MpGDH in the plasmid DNA retained by the colonies that formed, a recombinant plasmid in the form of pYE2C-Mp-C88A/E554D was acquired that encodes a double mutant in which cysteine at position 88 was substituted with alanine and glutamic acid at position 554 was substituted with aspartic acid.

Next, strain Inv-Sc was transformed and the acquired transformant strain (strain Sc-Mp-C88A/E554D) was cultured and purified in accordance with section (2) of the test example using pYE2C-Mp-C88A/E554D, followed by measuring specific activity (U/A280) and then calculating relative specific activity.

Continuing, the ratio of reactivity with D- xylose to reactivity with D-glucose (Xyl/Glc(%)) was measured based on the procedure of section (3) of the aforementioned test example.
Furthermore, in Table 2, "C88A/E554D" means that C (Cys) at position 88 is substituted with A (Ala) and E (Glu) at position 554 is substituted with D (Asp), and the slash "/" means that it has both substitutions.

[Table 2]

| Recombinant Plasmid | Primer Sequence Nos. | Relative Specific Activity | Xyl/Glc (%) |
|---|---|---|---|
| pYE2C-Mp (wild-type, comparative example) | - | 100 | 1.4 |
| pYE2C-Mp-C88A (present invention) | 3,4 | 119 | 1.2 |
| pYE2C-Mp-C88A/E554D (present invention) | 5,6 | 125 | 1.2 |

As shown in Table 2, specific activity was confirmed to improve as a result of combining the amino acid substitution of E554D after having introduced C88A into the amino acid sequence of SEQ ID NO: 1. Moreover, this double mutant was also determined to demonstrate a greater improvement in substrate specificity than the wild type.
As has been described above, the mutants of the present invention were determined to demonstrate improved specific activity in comparison with enzymes prior to introduction of a mutation. FAD-GDH having this property makes it possible to shorten measurement time by improving reactivity, reduce costs by reducing the amount of enzyme used, and bring about improvement of accuracy by reducing the level of measurement noise attributable to contaminants, and is expected to be able to provide a measurement method, measurement reagent, measurement kit and sensor that are more practical than measurement methods and measurement reagents using known enzymes for glucose measurement.
Furthermore, as is disclosed in the present description, the mutant enzymes demonstrating improved specific activity of the present invention are thought to be highly useful enzymes that are provided with high substrate specificity for glucose and are capable of accurately measuring D-glucose levels even under conditions of contamination by sugar compounds such as D-xylose in the same manner as the Mucor-derived FAD-GDH described in Japanese Patent No. 4648993 as previously discovered by the inventors of the present invention.

## Claims

1. A flavin-binding glucose dehydrogenase consisting of the amino acid sequence represented by SEQ ID NO: 1, or an amino acid sequence having a sequence identity of 90% or more with the amino acid sequence represented by SEQ ID NO: 1, and having one or more amino acid substitutions at positions corresponding to amino acids selected from the following (a) and/or (b), and having improved specific activity in comparison with prior to carrying out that substitution:

(a) the amino acid at position 88 in the amino acid sequence set forth in SEQ ID NO: 1,
(b) the amino acid at position 554 in the amino acid sequence set forth in SEQ ID NO: 1.

2. The flavin-binding glucose dehydrogenase according to claim 1, having an amino acid substitution selected from the following (c) and/or (d):

(c) the amino acid at the position corresponding to position 88 in the amino acid sequence set forth in SEQ ID NO: 1 is alanine,
(d) the amino acid at the position corresponding to position 554 in the amino acid sequence set forth in SEQ ID NO: 1 is aspartic acid.

3. The flavin-binding glucose dehydrogenase according to claim 2, wherein the amino acid at the position corresponding to the cysteine residue at position 88 is substituted with alanine.

4. The flavin-binding glucose dehydrogenase according to claim 2, wherein the amino acid at the position corresponding to the glutamic acid residue at position 554 is substituted with aspartic acid.

5. The flavin-binding glucose dehydrogenase according to claim 1, which has amino acid substitutions at the position corresponding to position 88 and the position corresponding to position 554 of SEQ ID NO: 1, and having improved specific activity in comparison with prior to carrying out those substitutions.

6. The flavin-binding glucose dehydrogenase according to claim 2, wherein the amino acid at the position corresponding to position 88 in the amino acid sequence set forth in SEQ ID NO: 1 is alanine and the amino acid at the position corresponding to position 554 in the amino acid sequence set forth in SEQ ID NO: 1 is aspartic acid.

7. The flavin-binding glucose dehydrogenase according to claim 6, wherein the amino acid at the position corresponding to the cysteine residue at position 88 is substituted with alanine and the amino acid at the position corresponding to the glutamic acid residue at position 554 is substituted with aspartic acid.

8. A flavin-binding glucose dehydrogenase gene encoding the flavin-binding glucose dehydrogenase according to any of claims 1 to 7.

9. Recombinant DNA comprising the flavin-binding glucose dehydrogenase gene according to claim 8 inserted into vector DNA.

10. A host cell introduced with the recombinant DNA according to claim 9.

11. A method for producing flavin-binding glucose dehydrogenase, comprising the following steps:

(a) a step for culturing the host cell according to claim 10,
(b) a step for expressing the flavin-binding glucose dehydrogenase gene contained in the host cell, and
(c) a step for isolating the flavin-binding glucose dehydrogenase from the culture.

12. A glucose measurement method using the flavin-binding glucose dehydrogenase according to any of claims 1 to 7.

13. A glucose assay kit containing the flavin-binding glucose dehydrogenase according to any of claims 1 to 7.

14. A glucose sensor containing the flavin-binding glucose dehydrogenase according to any of claims 1 to 7.

## Patentansprüche

1. Flavin-bindende Glucosedehydrogenase, die aus Folgendem besteht: der Aminosäuresequenz, die durch SEQ ID NO: 1 dargestellt wird, oder einer Aminosäuresequenz mit einer Sequenzidentität von 90% oder mehr mit der Aminosäuresequenz, die durch SEQ ID NO: 1 dargestellt wird, und mit einer oder mehreren Aminosäuresubstitutionen an Positionen, die Aminosäuren entsprechen, die aus den folgenden (a) und/oder (b) ausgewählt sind, und die eine verbesserte spezifische Aktivität im Vergleich zu dem Zustand vor der Durchführung dieser Substitution aufweisen:

(a) die Aminosäure an Position 88 in der Aminosäuresequenz gemäß SEQ ID NO: 1,
(b) die Aminosäure an Position 554 in der Aminosäuresequenz gemäß SEQ ID NO: 1.

2. Flavin-bindende Glucosedehydrogenase nach Anspruch 1 mit einer Aminosäuresubstitution, die aus den folgenden (c) und/oder (d) ausgewählt ist:

(c) die Aminosäure an der Position, die Position 88 in der in SEQ ID NO: 1 angegebenen Aminosäuresequenz entspricht, ist Alanin,
(d) die Aminosäure an der Position, die Position 554 in der in SEQ ID NO: 1 angegebenen Aminosäuresequenz entspricht, ist Asparaginsäure.

3. Flavin-bindende Glucosedehydrogenase nach Anspruch 2, wobei die Aminosäure an der Position, die dem Cysteinrest an Position 88 entspricht, durch Alanin substituiert ist.

4. Flavin-bindende Glucosedehydrogenase nach Anspruch 2, wobei die Aminosäure an der Position, die dem Glutaminsäurerest an Position 554 entspricht, durch Asparaginsäure substituiert ist.

5. Flavin-bindende Glucosedehydrogenase nach Anspruch 1, die Aminosäuresubstitutionen an der Position, die Position 88 entspricht, und der Position, die Position 554 von SEQ ID NO: 1 entspricht, aufweist und die eine verbesserte spezifische Aktivität im Vergleich zu dem Zustand vor der Durchführung dieser Substitutionen aufweist.

6. Flavin-bindende Glucosedehydrogenase nach Anspruch 2, wobei die Aminosäure an der Position, die Position 88 in der Aminosäuresequenz gemäß SEQ ID NO: 1 entspricht, Alanin ist und die Aminosäure an der Position, die Position 554 in der Aminosäuresequenz gemäß SEQ ID NO: 1 entspricht, Asparaginsäure ist.

7. Flavin-bindende Glucosedehydrogenase nach Anspruch 6, wobei die Aminosäure an der Position, die dem Cysteinrest an Position 88 entspricht, durch Alanin ersetzt ist, und wobei die Aminosäure an der Position, die dem Glutaminsäurerest an Position 554 entspricht, durch Asparaginsäure substituiert ist.

8. Flavin-bindendes Glucosedehydrogenase-Gen, das die Flavin-bindende Glucosedehydrogenase gemäß einem der Ansprüche 1 bis 7 codiert.

9. Rekombinante DNA, die das Flavin-bindende Glucosedehydrogenase-Gen nach Anspruch 8 umfasst, das in Vektor-DNA eingefügt ist.

10. Wirtszelle, die mit der rekombinanten DNA gemäß Anspruch 9 eingeführt wurde.

11. Verfahren zur Herstellung von Flavin-bindender Glucosedehydrogenase, das folgende Schritte umfasst:

(a) einen Schritt zum Kultivieren der Wirtszelle gemäß Anspruch 10,
(b) einen Schritt zur Expression des Flavin-bindenden Glucosedehydrogenase-Gens, das in der Wirtszelle enthalten ist, und
(c) einen Schritt zum Isolieren der Flavin-bindenden Glucosedehydrogenase aus der Kultur.

12. Glucosemessverfahren unter Verwendung der Flavin-bindenden Glucosedehydrogenase nach einem der Ansprüche 1 bis 7.

13. Glucose-Assay-Kit, das die Flavin-bindende Glucose-Dehydrogenase nach einem der Ansprüche 1 bis 7 umfasst.

14. Glucosesensor, der die Flavin-bindende Glucosedehydrogenase nach einem der Ansprüche 1 bis 7 umfasst.

## Revendications

1. Glucose déshydrogénase de liaison à la flavine, constituée de la séquence d'acides aminés représentée par SEQ ID n° : 1, ou d'une séquence d'acides aminés ayant une identité de séquence de 90 % ou plus avec la séquence d'acides aminés représentée par SEQ ID n° : 1, et ayant une ou plusieurs substitutions d'acides aminés à des positions correspondant à des acides aminés sélectionnés parmi (a) et/ou (b) suivants, et ayant une activité spécifique améliorée par rapport au cas avant de réaliser cette substitution :

(a) l'acide aminé à la position 88 dans la séquence d'acides aminés présentée dans SEQ ID n° : 1,
(b) l'acide aminé à la position 554 dans la séquence d'acides aminés présentée dans SEQ ID n° : 1.

**2.** Glucose déshydrogénase de liaison à la flavine selon la revendication 1, ayant une substitution d'acide aminé sélectionnée parmi (c) et/ou (d) suivants :

(c) l'acide aminé à la position correspondant à position 88 dans la séquence d'acides aminés présentée dans SEQ ID n° : 1 est l'alanine,
(d) l'acide aminé à la position correspondant à position 554 dans la séquence d'acides aminés présentée dans SEQ ID n° : 1 est l'acide aspartique.

**3.** Glucose déshydrogénase de liaison à la flavine selon la revendication 2, dans laquelle l'acide aminé à la position correspondant au résidu de cystéine à la position 88 est substitué avec l'alanine.

**4.** Glucose déshydrogénase de liaison à la flavine selon la revendication 2, dans laquelle l'acide aminé à la position correspondant au résidu d'acide glutamique à la position 554 est substitué avec l'acide aspartique.

**5.** Glucose déshydrogénase de liaison à la flavine selon la revendication 1, qui a des substitutions d'acides aminés à la position correspondant à la position 88 et la position correspondant à la position 554 de SEQ ID n° : 1, et ayant une activité spécifique améliorée par rapport au cas avant de réaliser ces substitutions.

**6.** Glucose déshydrogénase de liaison à la flavine selon la revendication 2, dans laquelle l'acide aminé à la position correspondant à la position 88 dans la séquence d'acides aminés présentée dans SEQ ID n° : 1 est l'alanine et l'acide aminé à la position correspondant à la position 554 dans la séquence d'acides aminés présentée dans SEQ ID n° : 1 est l'acide aspartique.

**7.** Glucose déshydrogénase de liaison à la flavine selon la revendication 6, dans laquelle l'acide aminé à la position correspondant au résidu de cystéine à la position 88 est substitué avec l'alanine et l'acide aminé à la position correspondant au résidu d'acide glutamique à la position 554 est substitué avec l'acide aspartique.

**8.** Gène de glucose déshydrogénase de liaison à la flavine encodant la glucose déshydrogénase de liaison à la flavine selon l'une quelconque des revendications 1 à 7.

**9.** ADN recombinant, comprenant le gène de glucose déshydrogénase de liaison à la flavine selon la revendication 8 inséré dans un ADN vecteur.

**10.** Cellule hôte dans laquelle l'ADN recombinant selon la revendication 9 est introduit.

**11.** Procédé pour produire une glucose déshydrogénase de liaison à la flavine, comprenant les étapes suivantes :

(a) une étape pour effectuer la culture de la cellule hôte selon la revendication 10,
(b) une étape pour exprimer le gène de glucose déshydrogénase de liaison à la flavine contenu dans la cellule hôte, et
(c) une étape pour isoler la glucose déshydrogénase de liaison à la flavine à partir de la culture.

**12.** Procédé de mesure de glucose, utilisant la glucose déshydrogénase de liaison à la flavine selon l'une quelconque des revendications 1 à 7.

**13.** Kit d'analyse de glucose, contenant la glucose déshydrogénase de liaison à la flavine selon l'une quelconque des revendications 1 à 7.

**14.** Capteur de glucose, contenant la glucose déshydrogénase de liaison à la flavine selon l'une quelconque des revendications 1 à 7.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2007289148 A
- JP 4494978 B
- WO 07139013 A
- JP 2008237210 A
- JP 4648993 B
- WO 12169512 A
- WO 1365770 A
- EP 2647703 A1
- JP 2010035448 A


### Non-patent literature cited in the description


- Pharmaceuticals and Medical Devices Safety Information. *Pharmaceutical and Food Safety Bureau, Ministry of Health, Labour and Welfare,* October 2004
- **T.C. BAK ; R. SATO.** Studies on the glucose dehydrogenase of Aspergillus oryzae: I. Induction of its synthesis by p-benzoquinone and hydroquinone. *Biochim. Biophys. Acta,* 1967, vol. 139, 265-276
- **T.C. BAK.** Studies on the glucose dehydrogenase of Aspergillus oryzae: II. Purification and physical and chemical properties. *Biochim. Biophys. Acta,* 1967, vol. 139, 277-293
- **T.C. BAK.** Studies on the glucose dehydrogenase of Aspergillus oryzae: III. General enzymatic properties. *Biochim. Biophys. Acta,* 1967, vol. 146, 317-327
- **T.C. BAK ; R. SATO.** Studies on the glucose dehydrogenase of Aspergillus oryzae: IV. Histidyl residue as an active site. *Biochim. Biophys. Acta,* 1967, vol. 146, 328-335
- *Techniques,* 1989, vol. 1, 11-15
- Current Protocols in Molecular Biology. Wiley Interscience, 1989
- *Chemistry Today,* 24 June 1989
- **KRAMER.** *Nucleic Acids Res.,* 1984, vol. 12, 9441
- *Methods Enzymol.,* 1987, vol. 154, 350
- *Gene,* 1985, vol. 37, 73
- **ECKSTEIN.** *Nucleic Acids Res.,* 1985, vol. 13, 8749
- *Nucleic Acids Res.,* 1985, vol. 13, 8765
- *Nucleic Acids Res.,* 1986, vol. 14, 9679
- **KUNKEL.** *Proc. Natl. Acad. Sci. U.S.A.,* 1985, vol. 82, 488
- *Methods Enzymol.,* 1987, vol. 154, 367
- *Technique,* 1989, vol. 1, 11
- *Methods Mol. Cell. Biol.,* 1995, vol. 5, 255-269
- *J. Microbiol. Methods,* 2003, vol. 55, 481-484
- *Mol. Gen. Genet.,* 1989, vol. 218, 99-104

SEQUENCE LISTING

<110> KIKKOMAN Corporation

<120> Flavin-Binding Glucose Dehydrogenase with Improved Specific Activity

<130> P2770A

<160> 6

<170> PatentIn version 3.5

<210> 1
<211> 641
<212> PRT
<213> Mucor prainii

<400> 1

```
Met Lys Ile Thr Ala Ala Ile Ile Thr Val Ala Thr Ala Phe Ala Ser
1               5                   10                  15

Phe Ala Ser Ala Gln Gln Asp Thr Asn Ser Ser Ser Thr Asp Thr Tyr
            20                  25                  30

Asp Tyr Val Ile Val Gly Gly Gly Val Ala Gly Leu Ala Leu Ala Ser
        35                  40                  45

Arg Ile Ser Glu Asn Lys Asp Val Thr Val Ala Val Leu Glu Ser Gly
    50                  55                  60

Pro Asn Ala Asn Asp Arg Phe Val Val Tyr Ala Pro Gly Met Tyr Gly
65                  70                  75                  80

Gln Ala Val Gly Thr Asp Leu Cys Pro Leu Ile Pro Thr Thr Pro Gln
                85                  90                  95

Glu Asn Met Gly Asn Arg Ser Leu Thr Ile Ala Thr Gly Arg Leu Leu
            100                 105                 110

Gly Gly Gly Ser Ala Ile Asn Gly Leu Val Trp Thr Arg Gly Gly Leu
        115                 120                 125

Lys Asp Tyr Asp Ala Trp Glu Glu Leu Gly Asn Pro Gly Trp Asn Gly
    130                 135                 140

Ala Asn Leu Phe Lys Tyr Phe Lys Lys Val Glu Asn Phe Thr Pro Pro
145                 150                 155                 160

Thr Pro Ala Gln Ile Glu Tyr Gly Ala Thr Tyr Gln Lys Ser Ala His
                165                 170                 175
```

Gly Lys Lys Gly Pro Ile Asp Val Ser Phe Thr Asn Tyr Glu Phe Ser
180 185 190

Gln Ser Ala Ser Trp Asn Ala Ser Leu Glu Thr Leu Asp Phe Thr Ala
195 200 205

Leu Pro Asp Ile Leu Asn Gly Thr Leu Ala Gly Tyr Ser Thr Thr Pro
210 215 220

Asn Ile Leu Asp Pro Glu Thr Val Gln Arg Val Asp Ser Tyr Thr Gly
225 230 235 240

Tyr Ile Ala Pro Tyr Thr Ser Arg Asn Asn Leu Asn Val Leu Ala Asn
245 250 255

His Thr Val Ser Arg Ile Gln Phe Ala Pro Lys Asn Gly Ser Glu Pro
260 265 270

Leu Lys Ala Thr Gly Val Glu Trp Tyr Pro Thr Gly Asn Lys Asn Gln
275 280 285

Lys Gln Ile Ile Lys Ala Arg Tyr Glu Val Ile Ile Ser Ser Gly Ala
290 295 300

Ile Gly Ser Pro Lys Leu Leu Glu Ile Ser Gly Ile Gly Asn Lys Asp
305 310 315 320

Ile Val Ser Ala Ala Gly Val Glu Ser Leu Ile Asp Leu Pro Gly Val
325 330 335

Gly Ser Asn Met Gln Asp His Val His Ala Ile Thr Val Ser Thr Thr
340 345 350

Asn Ile Thr Gly Tyr Thr Thr Asn Ser Val Phe Val Asn Glu Thr Leu
355 360 365

Ala Gln Glu Gln Arg Glu Glu Tyr Glu Ala Asn Lys Thr Gly Ile Trp
370 375 380

Ala Thr Thr Pro Asn Asn Leu Gly Tyr Pro Thr Pro Glu Gln Leu Phe
385 390 395 400

Asn Gly Thr Glu Phe Val Ser Gly Lys Glu Phe Ala Asp Lys Ile Arg
405 410 415

Asn Ser Thr Asp Glu Trp Ala Asn Tyr Tyr Ala Ser Thr Asn Ala Ser
420 425 430

```
Asn Val Glu Leu Leu Lys Lys Gln Tyr Ala Ile Val Ala Ser Arg Tyr
        435                 440                 445

Glu Glu Asn Tyr Leu Ser Pro Ile Glu Ile Asn Phe Thr Pro Gly Tyr
    450                 455                 460

Glu Gly Ser Gly Asn Val Asp Leu Gln Asn Asn Lys Tyr Gln Thr Val
465                 470                 475                 480

Asn His Val Leu Ile Ala Pro Leu Ser Arg Gly Tyr Thr His Ile Asn
                485                 490                 495

Ser Ser Asp Val Glu Asp His Ser Val Ile Asn Pro Gln Tyr Tyr Ser
            500                 505                 510

His Pro Met Asp Ile Asp Val His Ile Ala Ser Thr Lys Leu Ala Arg
        515                 520                 525

Glu Ile Ile Thr Ala Ser Pro Gly Leu Gly Asp Ile Asn Ser Gly Glu
    530                 535                 540

Ile Glu Pro Gly Met Asn Ile Thr Ser Glu Asp Asp Leu Arg Ser Trp
545                 550                 555                 560

Leu Ser Asn Asn Val Arg Ser Asp Trp His Pro Val Gly Thr Cys Ala
                565                 570                 575

Met Leu Pro Lys Glu Leu Gly Gly Val Val Ser Pro Ala Leu Met Val
            580                 585                 590

Tyr Gly Thr Ser Asn Leu Arg Val Val Asp Ala Ser Ile Met Pro Leu
        595                 600                 605

Glu Val Ser Ser His Leu Met Gln Pro Thr Tyr Gly Ile Ala Glu Lys
    610                 615                 620

Ala Ala Asp Ile Ile Lys Asn Phe Tyr Lys Thr Gln His Lys Asn Gln
625                 630                 635                 640

Asn
```

<210> 2
<211> 1926
<212> DNA
<213> Mucor prainii

<400> 2

```
atgaagatca cagctgccat tatcactgtt gccacagcat ttgcttcttt tgcttctgct     60
caacaagaca caaattcttc ctcaactgat acttatgatt atgttatcgt tggcggcggt    120
gtagctggtt tggctttggc tagtcgtatc tctgaaaaca aggatgtcac tgttgctgtt    180
ctcgagtccg gtcctaatgc caatgataga tttgttgttt atgctcctgg catgtatggc    240
caagctgttg gcactgatct ctgtcctctc attcctacta ctcctcaaga aaatatgggc    300
aacagaagtc tcacaatcgc tactggtaga ttgctcggtg gtggcagtgc tattaatggt    360
ctcgtttgga cccgtggtgg cttgaaggat tacgatgctt gggaggagct cggtaaccct    420
ggatggaacg gtgccaactt gttcaagtac tttaagaagg tcgaaaactt cactcctcct    480
actcctgccc aaattgaata cggcgctact tatcagaaaa gtgctcatgg caagaaggga    540
cctattgatg tctctttcac gaactacgag ttctctcaat ctgctagctg gaacgcctca    600
ctcgaaaccc ttgatttcac tgcacttcct gatatcttga acggtacttt ggccggttac    660
tctaccactc ccaacatttt ggaccctgag actgttcaac gtgttgattc ctatactggt    720
tacattgctc cttacactag ccgtaacaac ctcaatgttt tggccaacca taccgtctcc    780
cgcattcaat ttgctcccaa gaatggtagc gaacctctca aggctaccgg tgttgaatgg    840
tatcccactg gcaacaagaa tcaaaagcaa attatcaagg cccgttatga agttatcatc    900
tcatctggtg ccattggtag tcctaagctt ttggaaatct ctggtatcgg taataaggat    960
atcgtctctg ctgctggtgt cgagtccttg attgacttgc ctggcgttgg ttccaacatg   1020
caagatcacg ttcatgctat cactgtctct actaccaata ttactggcta tactaccaac   1080
agcgtctttg tcaatgaaac ccttgcccaa gaacaaagag aagaatatga agccaacaag   1140
actggtatct gggctactac tcccaacaac ctcggttatc ctacgcccga acaactcttc   1200
aatggcaccg aattcgtttc tggaaaggag tttgctgaca agattcgtaa ctctactgat   1260
gaatgggcca actattatgc ttccaccaac gcctccaatg tcgagttatt aaagaagcaa   1320
tatgctattg tcgcctctcg ttacgaagag aactacttgt ctcctattga aatcaacttc   1380
actcctggtt atgagggtag cggtaatgtc gatttgcaaa acaacaagta ccaaactgtc   1440
aaccatgtct tgattgctcc tttaagtcgt ggttatactc acattaactc ttctgatgtg   1500
gaggatcatt ctgtcattaa tccccaatac tactctcatc ctatggatat tgatgtccat   1560
atcgcttcca ctaaacttgc tcgcgaaatc atcactgcct ctcccggtct tggtgacatt   1620
aacagtggcg aaatcgaacc cggtatgaat attacttctg aagacgacct tagatcttgg   1680
ttgagtaata atgtccgttc tgactggcat cctgttggta cttgtgctat gcttcccaag   1740
gaattaggtg gtgttgtcag ccccgctctc atggtttacg gcacttccaa cttgcgtgtt   1800
gttgatgctt cgattatgcc cctcgaagtc tcttctcatt tgatgcaacc cacctacggt   1860
```

attgctgaga aggctgctga cattattaag aatttctaca agactcaaca caagaaccaa 1920

aattag 1926

<210> 3
<211> 30
<212> DNA
<213> Artificial Sequence

<220>
<223> Artificially Synthesized Primer Sequence

<400> 3
ggcactgatc tcgctcctct cattcctact 30

<210> 4
<211> 30
<212> DNA
<213> Artificial Sequence

<220>
<223> Artificially Synthesized Primer Sequence

<400> 4
attttcttga ggagtagtag gaatgagagg 30

<210> 5
<211> 30
<212> DNA
<213> Artificial Sequence

<220>
<223> Artificially Synthesized Primer Sequence

<400> 5
aatattactt ctgacgacga ccttagatct 30

<210> 6
<211> 30
<212> DNA
<213> Artificial Sequence

<220>
<223> Artificially Synthesized Primer Sequence

<400> 6
attattactc aaccaagatc taaggtcgtc 30